# EUROPEAN PATENT APPLICATION

(11) **EP 4 403 152 A1**
(43) Date of publication of application: **24.07.2024**
(21) Application number: 22911043.2
(22) Date of filing: 14.12.2022
(51) Int. Cl.: A61F 13/51, A61F 13/53, A61F 13/539

(54) **ABSORBENT ARTICLE**

(30) Priority: 20.12.2021 JP 2021206500; 20.12.2021 JP 2021206502
(71) Applicant: Unicharm Corporation, Ehime 799-0111 (JP)
(72) Inventor: BANDOU, Takeshi, Kanonji-shi, Kagawa 769-1602 (JP); MURAI, Takamasa, Kanonji-shi, Kagawa 769-1602 (JP); WATANABE, Sakiko, Kanonji-shi, Kagawa 769-1602 (JP); MIYAZAKI, Hirokazu, Kanonji-shi, Kagawa 769-1602 (JP); TODA, Haruki, Kanonji-shi, Kagawa 769-1602 (JP)
(74) Representative: Dolleymores
(86) International application number: PCT/JP2022/046093
(87) International publication number: WO 2023/120335

(57) **Abstract**

This absorbent article (1, 2) comprises a recycled sheet (8, 9, 38, 39) that contains recycled pulp in the amount of at least 15 wt%, the recycled pulp produced by recycling disposable absorbent articles. The absorbent article further comprises a composite sheet (Z1, Z2, Z3, Z4) in which the recycled sheet (8, 9, 38, 39) and a separate sheet (4, 5, 34, 35), which is adjacent to the recycled sheet in the direction of thickness of the absorbent article, are bonded to each other. When the composite sheet is stretched in the direction of the orientation of the fibers in the recycled sheet, the tensile force per unit width at which the recycled sheet (8, 9, 38, 39) of the composite sheet (Z1, Z2, Z3, Z4) starts to break is greater than the tensile force per unit width at which the separate sheet (4, 5, 34, 35) starts to break, when the separate sheet alone is stretched in the direction of the orientation of the fibers.

## Description

### FIELD

The present invention relates to an absorbent article.

### BACKGROUND

In recent years, there is a demand for efforts to achieve the Sustainable Development Goals (SDGs), and in response to this, efforts are being made to utilize resources effectively. For example, a technology is known for collecting pulp fibers and superabsorbent resin (superabsorbent polymer) from used absorbent articles and recycling them. In addition, of such material recycling, there is a demand to promote horizontal recycling, for example, in which pulp, which is the raw material, is collected from used diapers and is utilized to produce diapers again, as an example. For example, Patent Document 1 discloses a paper composition containing pulp and absorbent resin that have been collected from absorbent articles, and discloses recycled paper.

### [CITATION LIST]

### [PATENT LITERATURE]

[Patent Document 1] Japanese Patent Application Publication No. 2021-75819

### SUMMARY

### [TECHNICAL PROBLEM]

The recycled paper described in Patent Document 1 has increased strength by containing an absorbent resin molecular weight of which is reduced, and this makes it possible to use the recycled paper as absorbent sanitary paper, craft paper, copy paper, and the like for use in disposable diapers, menstrual hygiene products, and the like. However, in the case of using such recycled paper as a material for an absorbent article, it is required to have a material strength to the extent that damage of the material is prevented even when used for a long time. In particular, in a sheet material made from the recycled pulp that is obtained by collecting raw material pulp from used diapers and making it be able to reuse, the fiber length of pulp tends to be short due to a cleaning step in the recycling process, and there are cases where sheet material contain foreign substances. As a result, in the process of forming the recycled pulp into a sheet, the bonds between fibers weakens, and this causes a risk that the material strength is impaired. If the absorbent article in which a sheet material containing such recycled pulp is used for a long time, there is a risk that the sheet material is damaged.

The present invention was achieved in light of conventional problems such as that described above, an aspect of the present invention is to provide an absorbent article in which a sheet made using recycled pulp is not damaged even after long-time use and which is environment-friendly.

### [SOLUTION TO PROBLEM]

A main aspect of the present invention of achieving the above-described aspect is an absorbent article including: a recycled sheet containing 15 wt% or more of recycled pulp that is obtained by recycling a used absorbent article; and a composite sheet in which the recycled sheet and a separate sheet that is adjacent to the recycled sheet in a thickness direction of the absorbent article are joined, a tensile force per unit width at a timing when the recycled sheet starts to break in the composite sheet if the composite sheet is pulled in a fiber orientation direction of the recycled sheet being higher than a tensile force per unit width at a timing when the separate sheet starts to break if the separate sheet alone is pulled in the fiber orientation direction. Features of the present invention other than the above will become clear by reading the description of the present specification with reference to the accompanying drawings.

### [ADVANTAGEOUS EFFECT OF THE INVENTION]

According to the present invention, it is possible to provide an absorbent article in which a sheet made using recycled pulp is not damaged even after long-time use and which is environment-friendly.

### BRIEF DESCRIPTION OF DRAWINGS

FIG. 1 is a schematic plan view of a disposable diaper 1 in an unfolded and stretched state, which is an absorbent article according to the first embodiment, as viewed from the skin surface side.
FIG. 2 is a cross-sectional view taken along line A-A shown in FIG. 1.
FIG. 3 is a table showing the evaluation results of the tensile strength of sheets.
FIG. 4 shows an example of a load-elongation diagram in a tensile test of a composite sheet B.
FIG. 5 is a plan view showing the application range of the adhesive which joins a top-surface sheet 4 and a skin-side core-wrapping sheet 8.
FIG. 6 is a plan view showing the application range of the adhesive which joins a non-skin-side core-wrapping sheet 9 and a back-surface sheet 5.
FIG. 7 is a plan view further showing an adhesive with which an absorbent core 3 and a non-skin-side core-wrapping sheet 9 are joined, onto the plan view of FIG. 6.
FIG. 8 is a schematic plan view of an absorbent pad 2 in the unfolded and stretched state, which is an absorbent article according to the second embodiment, as viewed from the skin surface side.
FIG. 9 is an exploded perspective view of the main part of the absorbent pad 2 shown in FIG. 8.
FIG. 10 is a cross-sectional view taken along line B-B shown in FIG. 8.
FIG. 11 is a plan view of a second absorbent core 33b and a second core-wrapping sheet 39 of the absorbent pad 2 in the unfolded state.

### DESCRIPTION OF EMBODIMENTS

At least the following matters will be clear with the description of this specification and the attached drawings.

An absorbent article including: a recycled sheet containing 15 wt% or more of recycled pulp that is obtained by recycling a used absorbent article; and a composite sheet in which the recycled sheet and a separate sheet that is adjacent to the recycled sheet in a thickness direction of the absorbent article are joined, a tensile force per unit width at a timing when the recycled sheet starts to break in the composite sheet if the composite sheet is pulled in a fiber orientation direction of the recycled sheet being higher than a tensile force per unit width at a timing when the separate sheet starts to break if the separate sheet alone is pulled in the fiber orientation direction.

According to the absorbent article, by joining the recycled sheet and the separate sheet, the strength of the recycled sheet becomes a strength that the recycled sheet do not break unless there is applied a force greater than the tensile force at the timing when the separate sheet alone begins to break. Therefore, It is possible to improve the strength. Since such a recycled sheet having such strength is used for the absorbent article, it is possible to reduce the risk that the recycled sheet is damaged even if used for a long time.

An absorbent article including: a recycled sheet containing recycled pulp that is obtained by recycling a used absorbent article; and a composite sheet in which the recycled sheet and a separate sheet that is adjacent to the recycled sheet in a thickness direction of the absorbent article are joined, a content of the recycled pulp in the recycled sheet being less than 15 wt%, a tensile force per unit width at a timing when the recycled sheet starts to break in the composite sheet if the composite sheet is pulled in a fiber orientation direction of the recycled sheet is higher than a tensile force per unit width at a timing when the separate sheet starts to break if the separate sheet alone is pulled in the fiber orientation direction.

According to the absorbent article, by joining the recycled sheet and the separate sheet, the strength of the recycled sheet itself in the composite sheet where these sheets are joined becomes a strength that the recycled sheet do not break unless there is applied a force greater than the tensile force at the timing when the separate sheet alone begins to break. Therefore, It is possible to improve the strength of the recycled sheet. By reducing the content of the recycled pulp to less than 15 wt%, it is possible to maintain such strength of the recycled sheet, reducing the risk of the recycled sheet being damaged even if the recycled sheet in the absorbent article is used for a long time.

In such an absorbent article, it is desirable that in the composite sheet, the fiber orientation direction of the recycled sheet and the fiber orientation direction of the separate sheet are an identical direction, and that a maximum elongation of the separate sheet in the fiber orientation direction is lower than a maximum elongation in a direction perpendicular to the fiber orientation direction.

According to the absorbent article, compared to making the fiber orientation direction of the recycled sheet conform to the direction in which the maximum elongation is higher in the separate sheet, a force applied to the composite sheet is made less likely to break the separate sheet, making the recycled sheet further less likely to break in the case of making the fiber orientation direction of the recycled sheet conform to the direction in which the maximum elongation is lower in the separate sheet, that is, a direction where it is more difficult to elongate.

In such an absorbent article, it is desirable that, in the fiber orientation direction, a tensile force per unit width when a length of the separate sheet after elongation is 105% of the length of the separate sheet before elongation is higher than a tensile force per unit width when the recycled sheet starts to break when pulled in the fiber orientation direction.

According to the absorbent article, the separate sheet does not break even if it is elongated by 5%, and when compared to the recycled sheet alone, it does not break before the recycled sheet alone breaks. By joining a separate sheet having such strength and a recycled sheet as a material, it is possible to increase the strength of the recycled sheet in the composite sheet.

In such an absorbent article, it is desirable that the recycled sheet contains a thermoplastic resin.

According to the absorbent article, it is possible to reduce a risk that the recycled sheet containing the thermoplastic resin is damaged.

In such an absorbent article, it is desirable that the core-wrapping sheet and the separate sheet are thermally-fused.

According to the absorbent article, by thermally-fusing the thermoplastic resin of the recycled sheet and the thermoplastic resin of the separate sheet, it is possible to increase the strength of the recycled sheet in the composite sheet.

In such an absorbent article, it is desirable that the thermoplastic resin of the recycled sheet and the thermoplastic resin of the separate sheet are an identical type of thermoplastic resin.

According to the absorbent article, thermal-fusing of the identical type of the thermoplastic resin makes it possible to increase the strength of the recycled sheet in the composite sheet.

In such an absorbent article, it is desirable that, in the composite sheet, the recycled sheet and the separate sheet are joined with a hot-melt adhesive.

According to the absorbent article, by joining with the hot-melt adhesive, it is possible to further increase the tensile force at the timing when the recycled sheet in the composite sheet starts to break.

In such an absorbent article, it is desirable that the hot-melt adhesive is applied in a planar manner to at least a part.

According to the absorbent article, the strength of the recycled sheet can further increase in portions where the hot-melt adhesive is applied without gaps (solid-applied portion).

In such an absorbent article, it is desirable that the composite sheet has a non-joined region where the recycled sheet and the separate sheet are not joined with the hot-melt adhesive.

According to the absorbent article, if the hot-melt adhesive is applied completely without any gap to the entire surface, there is a risk that the composite sheet will become the composite sheet becomes stiff. However, by having non-joining region, the softness can be maintained and a balance between strength and hardness can be achieved.

In such an absorbent article, it is desirable that, in an unfolded state, the fiber orientation direction is a lengthwise direction of the absorbent article and a direction perpendicular to the lengthwise direction is a width direction, and that letting a region in the composite sheet where the recycled sheet and the separate sheet are joined with the hot-melt adhesive be a joining region, a total length of the joining region in the width direction is longer than a total length of the non-joining regions in the width direction.

According to the absorbent article, by increasing the region that is fixed with the hot-melt adhesive, it is possible to improve the strength of the composite sheet, making the recycled sheet further less likely to break.

In such an absorbent article, it is desirable that, in an unfolded state, the fiber orientation direction is a lengthwise direction of the absorbent article and a direction perpendicular to the lengthwise direction is a width direction, and that a length where the hot-melt adhesive is provided continuously in the composite sheet is longer in the lengthwise direction than in the width direction.

According to the absorbent article, in the recycled sheet the tensile strength in the lengthwise direction, which is the fiber orientation direction, is higher than the tensile strength in the width direction. Since the hot-melt adhesive is provided elongated continuously in the lengthwise direction, which is the fiber orientation direction, it is possible to make the recycled sheet further less likely to break.

In such an absorbent article, it is desirable that at least a part of a joining region where the recycled sheet and the separate sheet are joined with the hot-melt adhesive is provided over an entire range of the recycled sheet in the lengthwise direction.

According to the absorbent article, in the lengthwise direction, in which the tensile strength is high, since the joining region of the hot-melt adhesive exists over the entire range of the recycled sheet, it can make the recycled sheet further less likely to break.

In such an absorbent article, it is desirable that the absorbent article has an absorbent core, that the absorbent article has a skin-side composite sheet that is the composite sheet placed on the skin side in the thickness direction of the absorbent core, and a non-skin-side composite sheet that is the composite sheet placed on the non-skin side in the thickness direction of the absorbent core, that the recycled sheet of the skin-side composite sheet is a skin-side core-wrapping sheet that is located adjacent to the absorbent core on a skin side, and that the recycled sheet of the non-skin-side composite sheet is a non-skin-side core-wrapping sheet that is located adjacent to the absorbent core on a non-skin side.

According to the absorbent article, the absorbent core swells when it absorbs liquid, and therefore if the core-wrapping sheet is provided so as to wrap the absorbent core, the swelling of the absorbent core makes a force be applied to the core-wrapping sheet, causing a risk that the core-wrapping sheet on the side that wraps the absorbent core breaks. By arranging the core-wrapping sheet individually on the skin side and the non-skin side, it is possible to reduce the burden on the core-wrapping sheet and to reduce the risk of breakage.

In such an absorbent article, it is desirable that the separate sheet of the skin-side composite sheet is a liquid-permeable sheet member, that the separate sheet of the non-skin-side composite sheet is liquid-impermeable sheet member, and that a tensile force per unit width at a timing when the recycled sheet starts to break in the non-skin-side composite sheet if the non-skin-side composite sheet is pulled in a fiber orientation direction of the recycled sheet is higher than a tensile force per unit width at a timing when the recycled sheet starts to break in the skin-side composite sheet if the skin-side composite sheet is pulled in the fiber orientation direction of the recycled sheet.

According to the absorbent article, the tensile force at the timing when the recycled sheet starts to break in the composite sheet increases as the tensile force at the timing when the separate sheet to be joined starts to break increases. Since the non-skin-side composite sheet is joined to the liquid-impermeable sheet member (separate sheet), the recycled sheet of the non-skin-side composite sheet has a higher tensile force the timing of starting of breakage than the recycled sheet of the skin-side composite sheet. Therefore, compared to the case where the tensile force per unit width at the timing when the recycled sheet starts to break in the non-skin-side composite sheet is lower than the tensile force per unit width at the timing when the recycled sheet starts to break in the skin-side composite sheet, this makes it possible to further improve leakage prevention effect in the case where at the timing when the recycled sheet starts to break in the non-skin-side composite sheet is higher than the tensile force per unit width at the timing when the recycled sheet starts to break in the skin-side composite sheet.

In such an absorbent article, it is desirable that the separate sheet of the non-skin-side composite sheet is located adjacent to the non-skin-side core-wrapping sheet on the non-skin side, that the non-skin-side composite sheet has a non-joined region where the non-skin-side core-wrapping sheet and the separate sheet are not joined with a hot-melt adhesive, that the absorbent core and the non-skin-side core-wrapping sheet have a core joining region where the absorbent core and the non-skin-side core-wrapping sheet are joined to each other with the hot-melt adhesive, and that the non-joining region has a portion that overlaps the core joining region in a plan view of the absorbent article that is in an unfolded state.

According to the absorbent article, there is a risk that the strength of the non-joining region where the hot-melt adhesive is not applied decreases, but by having a portion that overlaps the core joining region where the absorbent core and the non-skin-side core-wrapping sheet are joined, it is possible to suppress the decrease in strength.

In such an absorbent article, it is desirable that the absorbent article includes a first absorbent core that contains at least a superabsorbent polymer and a second absorbent core that contains at least a superabsorbent polymer, that a mass ratio of the superabsorbent polymer contained in the first absorbent core is greater than a mass ratio of the superabsorbent polymer contained in the second absorbent core, that a first recycled sheet that is the recycled sheet is provided as a core-wrapping sheet that is arranged so as not to wrap the first absorbent core, and that a second recycled sheet that is the recycled sheet is provided as a core-wrapping sheet that is arranged so as to wrap the second absorbent core.

According to the absorbent article, since the core-wrapping sheet of the first absorbent core, which has a large amount of superabsorbent polymer, is made into a form that does not wrap the first absorbent core, this reduces the burden on the core-wrapping sheet of the first absorbent core, which has a large amount of the superabsorbent polymer and thereby is more likely to swell, making it possible to reduce the risk that the core-wrapping sheet (first recycled sheet) break.

In such an absorbent article, it is desirable that the recycled sheet contains a thermoplastic resin, that at least a part of the second recycled sheet has an overlapping region where portions of the recycled sheet overlap each other, in a plan view of the absorbent article that is in the unfolded state, and that, in the overlapping region, the portions of the recycled sheet are thermally-fused to each other.

According to the absorbent article, by thermally-fusing the portions of the recycled sheet, it is possible to increase the strength of the recycled sheet and to further reduce the risk of breakage.

### First embodiment

### Basic configuration of diaper 1

The following describes a tape-type disposable diaper for adults (hereinafter also referred to as diaper 1) by way of example of an absorbent article according to the first embodiment. However, the present invention is not limited to the above, and the absorbent article is also applicable to tape-type disposable diapers for infants, urine absorbing pads, menstrual hygiene products, and the like.

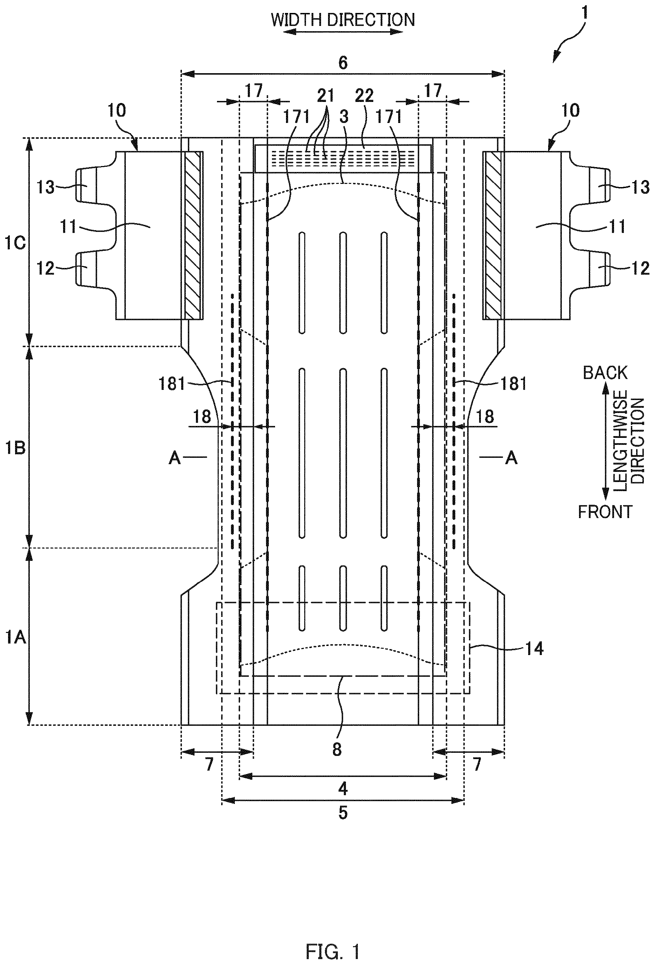

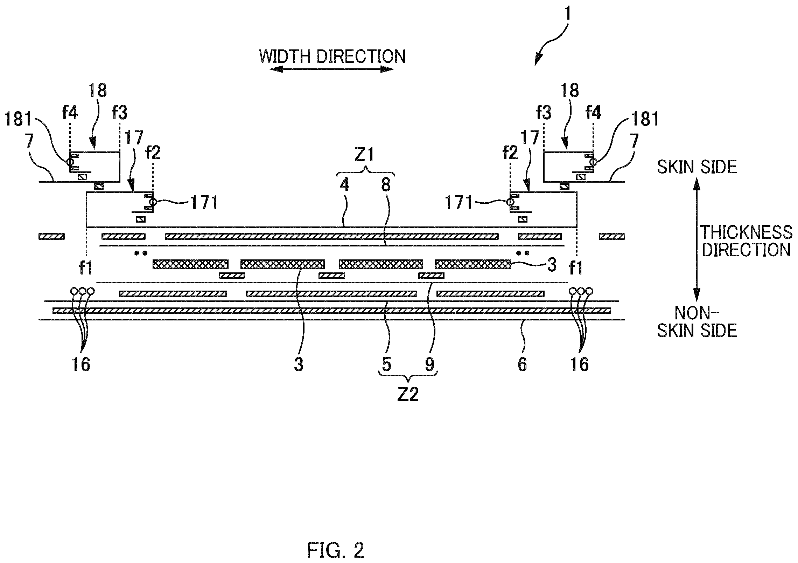

FIG. 1 is a schematic plan view of the disposable diaper 1 in an unfolded and stretched state, which is an absorbent article according to the first embodiment, as viewed from the skin surface side. FIG. 2 is a cross-sectional view taken along line A-A shown in FIG. 1.

The diaper 1 has a lengthwise direction, a width direction, and a thickness direction in the unfolded and stretched state, as shown in FIG. 1. In addition, the diaper 1 is divided into three regions in the lengthwise direction, and includes: a front waist portion 1A that is applied to the wearer's stomach side; a back waist portion 1C that is applied to the wearer's back side; and a crotch portion 1B that is located between front waist portion 1A and the back waist portion 1C in the lengthwise direction. Further, as shown in FIG. 2, the direction in which the constituent members of the diaper 1 are overlaid on each other is referred to as the thickness direction. In the thickness direction, the side that is in contact with the wearer is called the skin side, and the opposite side is called the non-skin side.

Note that the unfolded state of the diaper 1 refers to a state in which the diaper 1 is opened in the lengthwise direction and thus the diaper 1 is unfolded in a plan view. In addition, the "stretched state" of the diaper 1 refers to a state in which the entire diaper 1 (entire product) has been stretched to eliminate wrinkles, or more specifically has been stretched until the dimensions of constituent members of the diaper 1 (e.g., later-described inner and outer leak-proof-wall elastic members 171 and 181, leg elastic member 16, absorbent core 3, and the like) match or are close to the dimensions of the members on their own (in other words, the dimensions in a state where the elastic characteristics of the elastic member are not exhibited).

In addition, as shown in FIG. 2, the diaper 1 includes: an absorbent core 3; a top-surface sheet 4 provided on the skin side in the thickness direction with respect to the absorbent core 3; a back-surface sheet 5 and an exterior sheet 6 that are provided on the non-skin side in the thickness direction with respect to the absorbent core 3, and a pair of side sheets 7.

The top-surface sheet 4 only needs to be a liquid-permeable sheet, and examples thereof include an air-through nonwoven fabric sheet, a spunbond nonwoven fabric sheet and the like. The back-surface sheet 5 only needs to be a liquid-impermeable sheet, and examples thereof include a synthetic resin film and a hydrophobic SMS nonwoven fabric sheet and the like. Additionally, the top-surface sheet 4 and the back-surface sheet 5 contain thermoplastic resin (polyethylene (PE), polypropylene (PP), and the like).

The pair of side sheets 7 each are a sheet (e.g., nonwoven fabric sheet, and the like) located on the skin side of the top-surface sheet 4 and extending outward in the width direction from both side portions in the width direction. The exterior sheet 6 is a liquid-impermeable sheet located on the non-skin side in the thickness direction with respect to the back-surface sheet 5, and has a shape (that is, the external shape of the diaper 1) in which the center portion (crotch portion 1B) in the lengthwise direction is narrowed inward in the width direction.

For example, the absorbent core 3 can be obtained by molding liquid-absorbent fibers (e.g., pulp) containing SAP (superabsorbent polymer) into a predetermined shape. In addition, in the present embodiment, a liquid-permeable skin-side core-wrapping sheet 8 is arranged adjacent to the skin side of the absorbent core 3, and a liquid-permeable non-skin-side core-wrapping sheet 9 is arranged adjacent to the non-skin side of the absorbent core 3. The skin-side core-wrapping sheet 8 and the non-skin-side core-wrapping sheet 9 are constituted by tissue paper, for example, and the details thereof will be described later.

The diaper 1 also includes flap portions 10 extending outward in the width direction from the back waist portion 1C, as shown in FIG. 1. The flap portions 10 each include a base sheet 11, and a first fastening member 12 and a second fastening member 13 that are joined to the base sheet 11. The base sheet 11 is joined between the side sheet 7 and the exterior sheet 6. The first fastening member 12 and the second fastening member 13 are arranged spacing in the lengthwise direction from each other, and are configured to be able to fasten to a target portion 14 when putting on the diaper 1, the target portion 14 being provided in the front waist portion 1A. The first fastening member 12 and the second fastening member 13 can be a hook material, for example. Note that a configuration may be acceptable in which one fastening member is provided on each of the left and right sides. Moreover, a configuration may be acceptable in which the diaper 1 does not include the base sheet 11 of the flap portion 10, but the side sheet 7 and the exterior sheet 6 extend outward in the width direction, and the fastening members are joined onto the side sheet 7.

The diaper 1 has leg elastic members 16 that stretches and contracts in the lengthwise direction on both side portions in the width direction (see FIG. 2). Three leg elastic members 16 are provided on each side in the width direction with spacing from each other. More specifically, the leg elastic member 16 that is located farthest inside in the width direction is arranged between the top-surface sheet 4 and the back-surface sheet 5. The remaining two leg elastic members 16 are arranged between the side sheet 7 and the back-surface sheet 5. Note that the number of the leg elastic members 16 is not particularly limited.

Moreover, the diaper 1 has inner leak-proof gathers 17 and outer leak-proof gathers 18. The inner leak-proof gathers 17 and the outer leak-proof gathers 18 are configured to be able to rise up on the side of the wearer's skin surface, and are provided in pair on the left and right sides. The inner leak-proof gathers 17 are so-called inward-folding gathers, and the outer leak-proof gathers 18 are so-called outward-folding gathers. The inner leak-proof gathers 17 are each provided inside the corresponding outer leak-proof gather 18 and leg elastic members 16 in the width direction. Note that it is sufficient that at least a part of the inner leak-proof gather 17 is located inside the corresponding outer leak-proof gather 18 in the width direction. Moreover, the diaper 1 only needs to have at least the outer leak-proof gathers 18, and does not need to have the inner leak-proof gathers 17.

The inner leak-proof gather 17 is formed by folding the top-surface sheet 4, as shown in FIG. 2. Specifically, the inner leak-proof gather 17 is formed as follow: the top-surface sheet 4 is folded inward from the outside in the width direction and toward the skin side at a folding line f1 shown in FIG. 2, and further the top-surface sheet 4 is folded back outward from the inside in the width direction and toward the non-skin side at a folding line f2, and an inner leak-proof elastic member 171 that stretches and contracts in the lengthwise direction is provided in the folded-back portion. Examples of the inner leak-proof elastic member 171 include elastic strings.

The outer leak-proof gather 18 is formed by folding the side sheet 7, as shown in FIG. 2. Specifically, the outer leak-proof gather 18 is formed as follow: the side sheet 7 is folded outward from the inside in the width direction and toward the skin side at the folding line f3 shown in FIG. 2, and further the side sheet 7 is folded back inward from the outside in the width direction and toward the non-skin side at the folding line f4, and an outer leak-proof elastic member 181 that stretches and contracts in the lengthwise direction is provided in the folded-back portion. Examples of the outer leak-proof elastic member 181 include elastic strings. The inner leak-proof gathers 17 and the outer leak-proof gathers 18 can suppress lateral leakage of excrement.

In the back waist portion 1C of the diaper 1, a waist gather 22 formed by a plurality of waist elastic members 21 is arranged in the end in the lengthwise direction and in the center in the width direction. Due to the waist gather 22, the diaper 1 deforms to fit the shape of the wearer's waist when putting on, and also is suppressed diaper 1 from shifting.

### Skin-side core-wrapping sheet 8 and non-skin-side core-wrapping sheet 9

The skin-side core-wrapping sheet 8 and the non-skin-side core-wrapping sheet 9 in the present embodiment are a recycled sheet that contains recycled pulp which is obtained by recycling used disposable absorbent articles (e.g., used disposable diapers). Generally, in the absorbent article, the material is required to be strong enough to prevent the absorbent core from being exposed to the outside after long periods of use. On the other hand, concerning a sheet containing recycled pulp that is obtained by recovering raw material pulp from collected used disposable diapers and making it be able to reuse by the recycling process, such a sheet has the characteristic that the fiber length of the recycled pulp becomes shorter during a cleaning step. As a result, the strength of the material is impaired, and in the case where, for example, a sheet containing the recycled pulp is used as the core-wrapping sheet, there is a risk that the core-wrapping sheet is damaged when the absorbent article is used for a long time and thereby the absorbent core is exposed.

In this regard, first, it is preferable that the skin-side core-wrapping sheet 8 and the non-skin-side core-wrapping sheet 9, which are recycled sheets containing the recycled pulp, contain 15 wt% or more of the recycled pulp. The diaper 1 includes a composite sheet in which the recycled sheet and a separate sheet that is adjacent to the recycled sheet in the thickness direction of the diaper 1 are joined. Specifically, the separate sheet for the skin-side core-wrapping sheet 8 (recycled sheet) is the top-surface sheet 4, and the skin-side core-wrapping sheet 8 and the top-surface sheet 4 are joined to form a composite sheet. That is, the diaper 1 has a skin-side composite sheet Z1 that is a composite sheet placed on the skin side of the absorbent core 3 in the thickness direction. Further, the separate sheet for the non-skin-side core-wrapping sheet 9 (recycled sheet) is the back-surface sheet 5, and the non-skin-side core-wrapping sheet 9 and the back-surface sheet 5 are joined to form a composite sheet. That is, the diaper 1 has a non-skin-side composite sheet Z2 that is a composite sheet placed on the non-skin side of the absorbent core 3 in the thickness direction.

Here, in order to confirm whether there increases the material strength of a composite sheet in which a separate sheet and the recycled sheet containing the recycled pulp, which has a short fiber length, a test was conducted using the following method for each of the recycled sheet, separate sheet, and composite sheet.

### Evaluation method

The followings are prepared as materials for evaluation:
1) Recycled sheet containing 15 wt% of the recycled pulp (skin-side core-wrapping sheet 8);
2) As an example of the top-surface sheet 4 (separate sheet), polypropylene spunbond nonwoven fabric sheet (PPSB);
3) As an example of the top-surface sheet 4 (separate sheet), air-through nonwoven fabric sheet;
4) As an example of the skin-side composite sheet Z1, composite sheet A made by laminating the sheets 1) and 2) described above with a hot-melt adhesive (the application amount of the adhesive is 4 g/m² and is applied in a spiral pattern); and
5) As an example of the skin-side composite sheet Z1, composite sheet B made by laminating the sheets 1) and 3) described above with a hot-melt adhesive (the application amount of the adhesive is 4 g/m² and is applied in a spiral pattern) . Each of the sheets 1) to 5) described above was cut out to a size of 25 mm x 100 mm, and five samples of each sheet were produced. Note that, in order to measure the strength of each sheet in a fiber orientation direction, the length of the sheet in the fiber orientation direction was set to 100 mm. In the case where the material is a nonwoven fabric sheet, the constituent fibers are arranged in the direction in which the nonwoven fabric web is conveyed (MD direction) when manufacturing the nonwoven fabric web, for example, and accordingly the fiber orientation direction means the direction along which the fibers of such material extend. The tensile strength factor of the material in the orientation direction of the constituent fibers (MD direction of web) is originally higher than the tensile strength factor in the direction perpendicular to the orientation direction of the constituent fibers (CD direction of web). That is, the fiber orientation direction is also the direction, of the lengthwise direction and the width direction of the diaper 1, whichever has the higher strength of the material. In the present embodiment, in the unfolded state shown in FIG. 1, the fiber orientation direction is the lengthwise direction of the diaper 1, and the direction perpendicular to the orientation direction of the constituent fibers is the width direction.

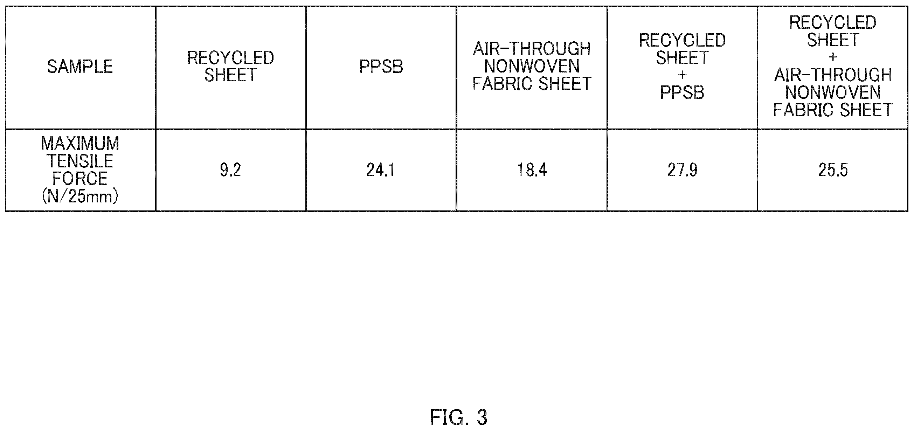

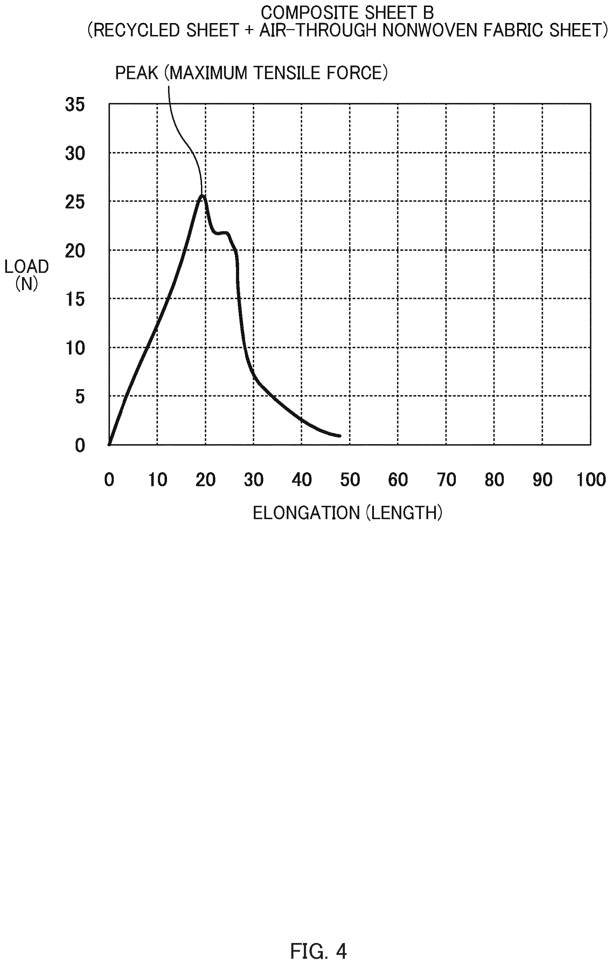

Then, using a tensile tester (autograph tensile tester AGS-1KNG manufactured by Shimadzu Corporation, and the like), the sample was pulled in the fiber orientation direction at a speed of 100 mm/min to measure the tensile strength. The same measurement was performed five times for each sample to calculate the respective maximum tensile force, and the average thereof was taken as the value of the maximum tensile force (tensile strength) for each sheet 1) to 5) . FIG. 3 is a table showing the evaluation results of the tensile strength of the sheets. Note that the width of the sheets is set to 25 mm as a standard, and therefore the denominator of the unit of maximum tensile force is 25 mm. Further, FIG. 4 shows an example of a load-elongation diagram in a tensile test of the above-mentioned composite sheet B. Among five measurements made using samples of the composite sheet B, as an example, the diagram in FIG. 4 shows the result of the sample whose maximum tensile force is close to the average value. Note that the peak (maximum point) shown in the diagram of FIG. 4 is the maximum tensile force, and this peak is the point at which the sheet begins to break. In reality, the breakage is completed after the point of maximum tensile force.

Referring to FIG. 3, the maximum tensile force of the recycled sheet alone, which is the sheet 1), was 9.2 N/25mm. On the other hand, the maximum tensile force of the composite sheet A made by joining the recycled sheet and PPSB, which is the sheet 4), was 27.9 N/25mm, and the maximum tensile force of composite sheet B made by joining the recycled sheet and the air-through nonwoven fabric sheet, which is the sheet 5) was 25.5 N/25mm. That is, in both cases, the strength increased. The maximum tensile force of the composite sheet A is higher than the maximum tensile force (24.1 N/25mm) of PPSB alone, which is the sheet 2), that is, its separate sheet. Also, the maximum tensile force of the composite sheet B is higher than the maximum tensile force (18.4 N/25mm) of the air-through nonwoven fabric alone, which is the sheet 3), that is, its separate sheet.

Here, the maximum tensile force per unit width (25 mm) of each sheet is the tensile force per unit width at which the sheet begins to break, i.e., the tensile force per unit width at which the recycled sheet in each of the composite sheets A and B starts to break. Therefore, from the above-described test, concerning the tensile force per unit width at the timing when the recycled sheet (skin-side core-wrapping sheet 8) of the composite sheet (skin-side composite sheet Z1) starts to break in the case of pulling the composite sheet in the fiber orientation direction of the recycled sheet, concerning the tensile force per unit width at the timing when the separate sheet (top-surface sheet 4) starts to break in the case of pulling the separate sheet alone in the fiber orientation direction, it can be said that the former tensile force is higher than the latter tensile force. In other words, by joining the recycled sheet (skin-side core-wrapping sheet 8) and the separate sheet (top-surface sheet 4), the strength of the recycled sheet (skin-side core-wrapping sheet 8) becomes a strength that the recycled sheet do not break unless there is applied a force greater than the tensile force at the timing when the separate sheet (top-surface sheet 4) starts to break. Since such a recycled sheet having such strength is used for the diaper 1, even if the wearer uses it for a long time, it is possible to reduce a risk of the recycled sheet being damaged.

Furthermore, the following can be said about the strength of the recycled sheet (non-skin-side core-wrapping sheet 9) in the non-skin-side composite sheet Z2. As mentioned above, in the present embodiment, the separate sheet of the skin-side composite sheet Z1 (top-surface sheet 4) is a liquid-permeable sheet member, and the separate sheet of the non-skin-side composite sheet Z2 (back-surface sheet 5) is a liquid-impermeable sheet member. Therefore, concerning the tensile force per unit width at the timing when the recycled sheet (non-skin-side core-wrapping sheet 9) starts to break in the non-skin-side composite sheet Z2 in the case of pulling the non-skin-side composite sheet Z2 in the fiber orientation direction of the recycled sheet, concerning the tensile force per unit width at the timing when the recycled sheet (skin-side core-wrapping sheet 8) starts to break in the skin-side composite sheet Z1 in the case of pulling the skin-side composite sheet Z1 in the fiber orientation direction of the recycled sheet, the former tensile force is higher than the latter tensile force. In other words, the tensile force at the timing when the recycled sheet in each composite sheet starts to break increases as the tensile force at the timing when the separate sheet to be joined starts to break increases. The non-skin-side composite sheet Z2 is joined to the liquid-impermeable separate sheet (back-surface sheet 5) such as a synthetic resin film, and therefore the recycled sheet of the non-skin-side composite sheet Z2 (non-skin-side core-wrapping sheet 9) has a higher tensile force at the timing of starting of breakage than the recycled sheet of the skin-side composite sheet Z1 (skin-side core-wrapping sheet 8). That is, the non-skin-side core-wrapping sheet 9 in the non-skin-side composite sheet Z2 is less likely to break than the skin-side core-wrapping sheet 8 in the skin-side composite sheet Z1. Therefore, compared to the case where the tensile force per unit width at the timing when the non-skin-side core-wrapping sheet 9 starts to break in the non-skin-side composite sheet Z2 is lower than the tensile force per unit width at the timing when the skin-side core-wrapping sheet 8 starts to break in the skin-side composite sheet Z1, this makes it possible to further improve leakage prevention effect in the case where the tensile force per unit width at the timing when the non-skin-side core-wrapping sheet 9 starts to break in the non-skin-side composite sheet Z2 is higher than the tensile force per unit width at the timing when the skin-side core-wrapping sheet 8 starts to break in the skin-side composite sheet Z1.

In addition, the recycled sheets (skin-side core-wrapping sheet 8 and non-skin-side core-wrapping sheet 9) contain a thermoplastic resin in addition to the pulp fiber as the liquid-absorbent fiber. Examples of the thermoplastic resin include polyethylene (PE) and polypropylene (PP).

In addition, in the present embodiment, the skin-side core-wrapping sheet 8 and the top-surface sheet 4 are joined to form the skin-side composite sheet Z1, which is a composite sheet. But as a modified example of the diaper 1, it is also conceivable that another sheet material such as an auxiliary sheet is arranged between the skin-side core-wrapping sheet 8 and the top-surface sheet 4. However, the composite sheet in the present embodiment is formed by joining the recycled sheet (skin-side core-wrapping sheet 8) with the separate sheet that can prevent the contents of the absorbent core 3 from leaking out due to damage, and therefore it is preferable that the separate sheet is not an auxiliary sheet or the like, but is the top-surface sheet 4.

In addition, in the skin-side composite sheet Z1, the fiber orientation direction of the recycled sheet (skin-side core-wrapping sheet 8) and the fiber orientation direction of the separate sheet (top-surface sheet 4) are the same direction. The maximum elongation (maximum elongation rate) in the fiber orientation direction (the lengthwise direction) is lower than the maximum elongation in the direction perpendicular to the fiber orientation direction (the width direction). The direction perpendicular to the fiber orientation direction is a direction in which the tensile strength factor is low and in which the separate sheet (top-surface sheet 4) is likely to stretch. If the fiber orientation direction of the recycled sheet is made conform to such a direction in which the separate sheet is likely to stretch, i.e. the width direction, this increase a risk that, when the separate sheet is pulled in the same direction (width direction), the recycled sheet that is joined to the separate sheet breaks. Therefore, compared to making the fiber orientation direction of the skin-side core-wrapping sheet 8 conform to the direction in which the maximum elongation is higher in the top-surface sheet 4, a force applied to the skin-side composite sheet Z1 is made less likely to break the top-surface sheet 4, making the skin-side core-wrapping sheet 8 further less likely to break in the case of making the fiber orientation direction of the skin-side core-wrapping sheet 8 conform to the direction where the maximum elongation is lower in the top-surface sheet 4, that is, the direction where it is more difficult to elongate.

In addition, the separate sheets (in the present embodiment, the top-surface sheet 4 and the back-surface sheet 5) themselves have the following strength. In the fiber orientation direction, concerning the tensile force per unit width when the length of the elongated separate sheet reaches to 105% of the length of the separate sheet before elongation, and concerning the tensile force per unit width at the timing when the recycled sheet (skin-side core-wrapping sheet 8 or non-skin-side core-wrapping sheet 9) starts to break if the recycled sheet alone is pulled in the fiber orientation direction, the former tensile force is higher than the latter tensile force. That is, from the above-mentioned test results for measuring tensile strength (FIG. 3), the tensile force per unit width (25 mm) at the timing when the recycled sheet starts to break is 9.2 N/25mm. And, this means that, when pulling a polypropylene spunbond nonwoven fabric sheet and an air-through nonwoven fabric sheet, which fall under the separate sheet, in the fiber orientation direction and the length after elongation becomes 105% of the length before elongation, the tensile force per unit width is higher than 9.2 N/25mm. In other words, the separate sheet (top-surface sheet 4 or back-surface sheet 5) does not break even if it is elongated by 5%, and even when compared to the recycled sheet (skin-side core-wrapping sheet 8 or non-skin-side core-wrapping sheet 9) alone, it does not break before the recycled sheet alone breaks. By joining a separate sheet having such strength and a recycled sheet as a material, it is possible to increase the strength of the recycled sheet in the skin-side composite sheet Z1 and the non-skin-side composite sheet Z2.

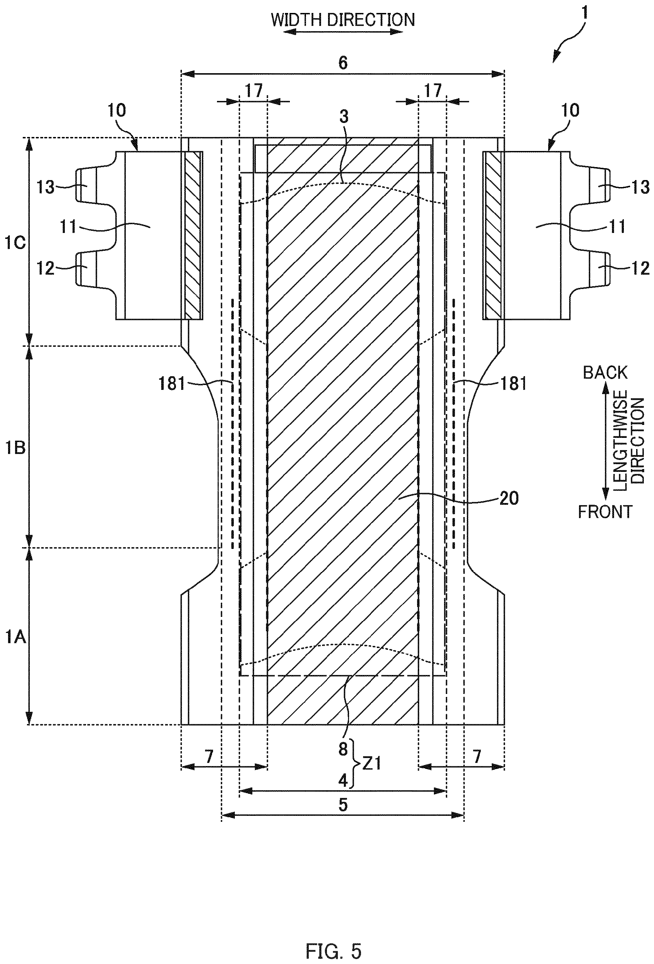

FIG. 5 is a plan view showing the application range of the adhesive which joins the top-surface sheet 4 and the skin-side core-wrapping sheet 8. In FIG. 5, the application region 20 of the adhesive (the downward-left hatched portion in the center in the width direction) is shown with the top-surface sheet 4 and the skin-side core-wrapping sheet 8 seeing through. As shown in the figure, in the skin-side composite sheet Z1 formed by the top-surface sheet 4 and the skin-side core-wrapping sheet 8, the skin-side core-wrapping sheet 8 and the top-surface sheet 4 are joined with a hot-melt adhesive applied in a planar manner. This makes it possible to further increase the tensile force at the timing when the skin-side core-wrapping sheet 8 (recycled sheet) starts to break in the skin-side composite sheet Z1.

Further, as shown in FIG. 5, the hot-melt adhesive is applied in a planar manner in the application region 20. This makes it possible to further increase the strength of the skin-side core-wrapping sheet 8 (recycled sheet) in the portion where the hot-melt adhesive is applied in a planar manner (application region 20), that is, in the portion where the hot-melt adhesive is applied without gaps.

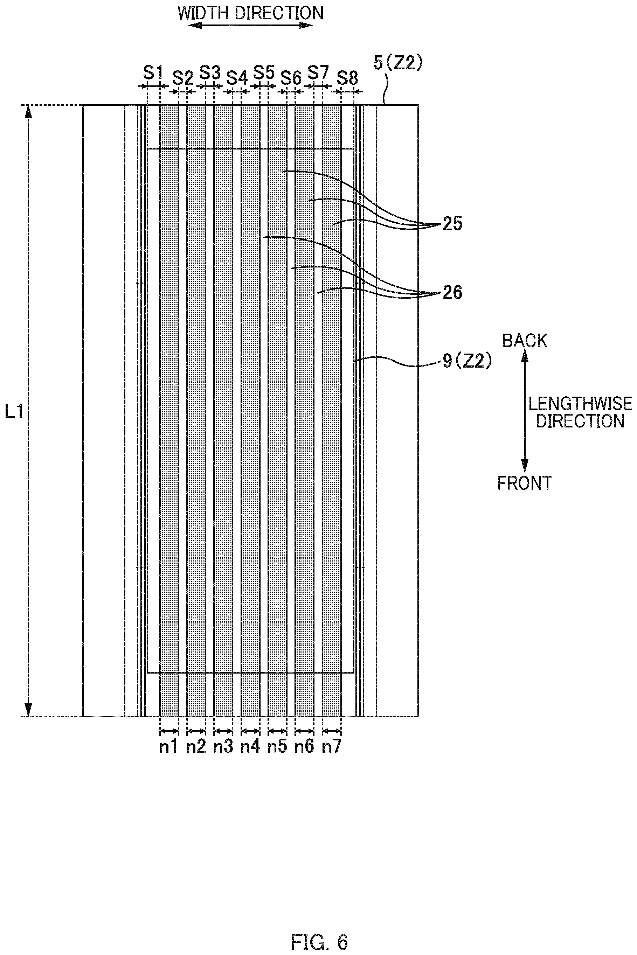

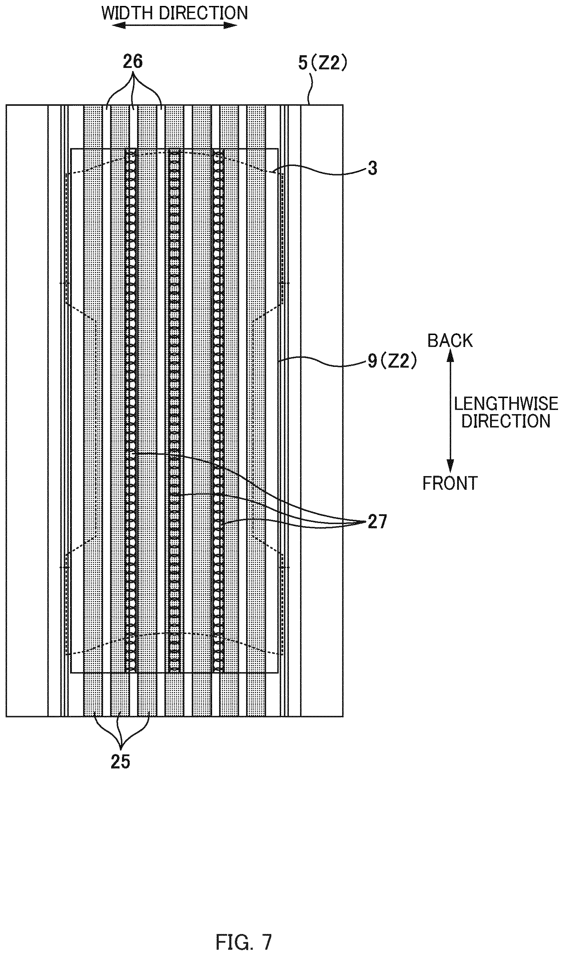

FIG. 6 is a plan view showing the application range of the adhesive which joins the non-skin-side core-wrapping sheet 9 and the back-surface sheet 5. FIG. 7 is a plan view further showing the adhesive with which the absorbent core 3 and the non-skin-side core-wrapping sheet 9 are joined, onto the plan view of FIG. 6. In both of FIGS. 6 and 7, the diaper 1 that is in the unfolded state is shown in plan view, and for convenience of explanation, the constituent materials are limited. In FIG. 6, the application range of the adhesive is shown with the non-skin-side core-wrapping sheet 9 and the back-surface sheet 5 seeing through. Also, in FIG. 7, the application range of the adhesive is shown with the non-skin-side core-wrapping sheet 9, the back-surface sheet 5, and the absorbent core 3 seeing through.

In the present embodiment, as shown in FIG. 6, in the non-skin-side composite sheet Z2 composed of the non-skin-side core-wrapping sheet 9 and the back-surface sheet 5, the non-skin-side core-wrapping sheet 9 (recycled sheet) and the back-surface sheet 5 (separate sheet) are joined with the hot-melt adhesive. Specifically, the non-skin-side composite sheet Z2 has a plurality of joining regions 25 where the non-skin-side core-wrapping sheet 9 and the back-surface sheet 5 are joined with the hot-melt adhesive. In the present embodiment, seven joining regions 25 are provided spacing in the width direction and extending along the lengthwise direction, but the number of the joining regions 25 is not limited to this. Examples of the application pattern of the hot-melt adhesive include an Ω pattern, a spiral pattern, and a solid coating pattern in which the adhesive is applied to the application target region without any gaps, but the present invention is not limited to these. In addition, in the case where the joining region 25 is formed by the hot-melt adhesive being applied, for example, in a circular application pattern, the width of the joining region 25 (later-described n1, n2, and the like) is not the width of the line which draws the circular pattern, but the diameter of the circular pattern. By making the joining with the hot-melt adhesive in this way, it is possible to further increase the tensile force at the timing when the non-skin-side core-wrapping sheet 9 (recycled sheet) starts to break in the non-skin-side composite sheet Z2.

Further, as shown in FIG. 6, the non-skin-side composite sheet Z2 has non-joining regions 26 where the non-skin-side core-wrapping sheet 9 and the back-surface sheet 5 are not joined with the hot-melt adhesive. Similarly, in the skin-side composite sheet Z1, the region excluding the application region 20 in the skin-side core-wrapping sheet 8 shown in FIG. 5 becomes a non-joining region. For example, if the hot-melt adhesive is applied completely without any gaps to the entire region where the non-skin-side core-wrapping sheet 9 and the back-surface sheet 5 overlap, there is a risk that the non-skin-side composite sheet Z2 becomes stiff. However, by having the non-joining regions 26, the softness can be maintained and a balance between strength and hardness can be achieved.

Here, as shown in FIG. 6, letting the lengths of the joining regions 25 in the width direction be n1, n2, ... n7, letting the lengths of the non-joining regions 26 in the width direction be S1, S2, ... S8, in the present embodiment, the total length of the joining regions 25 in the width direction in the non-skin-side composite sheet Z2 (n1 + n2 + n3 + n4 + n5 + n6 + n7) is longer than the total length of the non-joining regions 26 in the width direction (S1 + S2 + S3 + S4 + S5 + S6 + S7 + S8). In other words, in the non-skin-side composite sheet Z2, the region that is fixed with the hot-melt adhesive is larger. By increasing the number of non-joining regions 25 in this way, it is possible to improve the strength of the non-skin-side composite sheet Z2, making the non-skin-side core-wrapping sheet 9 (recycled sheet) in the non-skin-side composite sheet Z2 further less likely to break.

In addition, the length L1 of a portion in which hot-melt adhesive is provided continuously in the lengthwise direction of the non-skin-side composite sheet Z2 (i.e. the length of the joining regions 25 in the lengthwise direction) is longer than the length of a portion in which hot-melt adhesive is provided continuously in the width direction (any one of the lengths n1 to n7). For the non-skin-side core-wrapping sheet 9 (recycled sheet), the tensile strength in the lengthwise direction, which is the fiber orientation direction, is higher than the tensile strength in the width direction. Since the hot-melt adhesive is provided elongated continuously in the lengthwise direction, which is the fiber orientation direction, it is possible to make the non-skin-side core-wrapping sheet 9 further less likely to break.

Further, as shown in FIG. 5, the joining region where the skin-side core-wrapping sheet 8 and the top-surface sheet 4 are joined with the hot-melt adhesive (application region 20) is provided over the entire range of the skin-side core-wrapping sheet 8 in the lengthwise direction. Similarly, as shown in FIG. 6, the joining regions 25 where the non-skin-side core-wrapping sheet 9 and the back-surface sheet 5 are joined with the hot-melt adhesive are each provided over the entire range of the non-skin-side core-wrapping sheet 9 in the lengthwise direction. Note that it is sufficient that at least a part of the joining region 25 is provided over the entire range of the recycled sheet in the lengthwise direction. In the lengthwise direction, in which the tensile strength is high, since the joining regions (20, 25) of the hot-melt adhesive exist the entire range of the recycled sheets (skin-side core-wrapping sheet 8 and non-skin-side core-wrapping sheet 9) in the lengthwise direction, it can make the recycled sheets further less likely to break.

In the present embodiment, as mentioned above, the skin-side core-wrapping sheet 8, which is a recycled sheet of the skin-side composite sheets Z1 placed on the skin side in the thickness direction of the absorbent core 3, is placed adjacent to the absorbent core 3 on the skin side. Further, the non-skin-side core-wrapping sheet 9, which is a recycled sheet of the non-skin-side composite sheet Z2 placed on the non-skin side in the thickness direction of the absorbent core 3, is placed adjacent to the absorbent core 3 on the non-skin side. The absorbent core 3 swells when it absorbs liquid, and therefore if the core-wrapping sheet is provided so as to wrap around the absorbent core 3, the swelling of the absorbent core 3 makes a force be applied to the core-wrapping sheet, causing a risk that the core-wrapping sheet on the side that wraps the absorbent core 3 breaks. In the present embodiment, by arranging the core-wrapping sheets (skin-side core-wrapping sheet 8 and non-skin-side core-wrapping sheet 9) individually on the skin side and the non-skin side of the absorbent core 3, it is possible to reduce the burden on the core-wrapping sheet and to reduce the risk of breakage.

As shown in FIG. 7, the absorbent core 3 and the non-skin-side core-wrapping sheet 9 have core joining regions 27 where they are joined to each other with a hot-melt adhesive. In the present embodiment, three core joining regions 27 are provided spacing in the width direction and extending along the lengthwise direction, but the number of the core joining regions 27 is not limited to this. The non-joining regions 26 where the non-skin-side core-wrapping sheet 9 and the back-surface sheet 5 are not joined with the hot-melt adhesive has a portion that overlaps the core joining region 27 in a plan view. There is a risk that the strength of the non-joining region 26 where the hot-melt adhesive is not applied decreases, but by having a portion that overlaps the core joining region 27, it is possible to suppress the decrease in strength of the recycled sheet of the non-skin-side composite sheet Z2 (non-skin-side core-wrapping sheet 9).

### Second embodiment

The following describes an absorbent pad 2 that absorbs excrement such as urine and feces by way of example of an absorbent article according to the second embodiment.

### Basic structure of absorbent pad 2

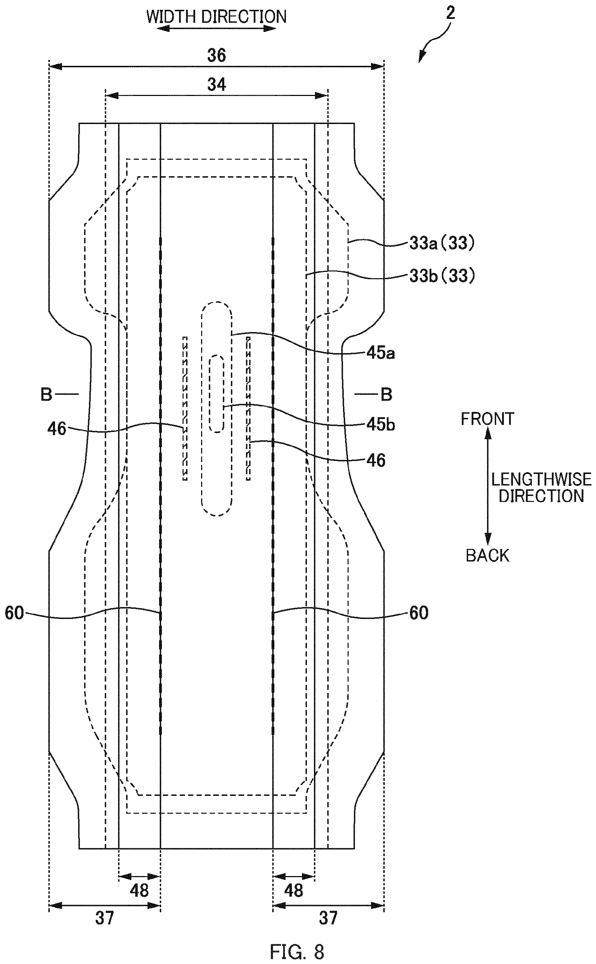

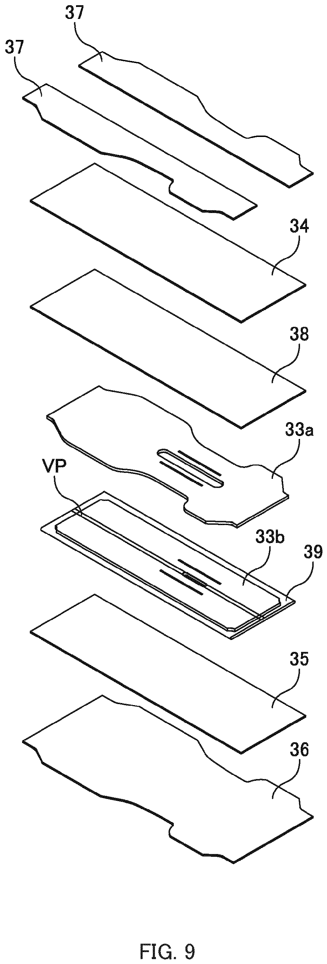

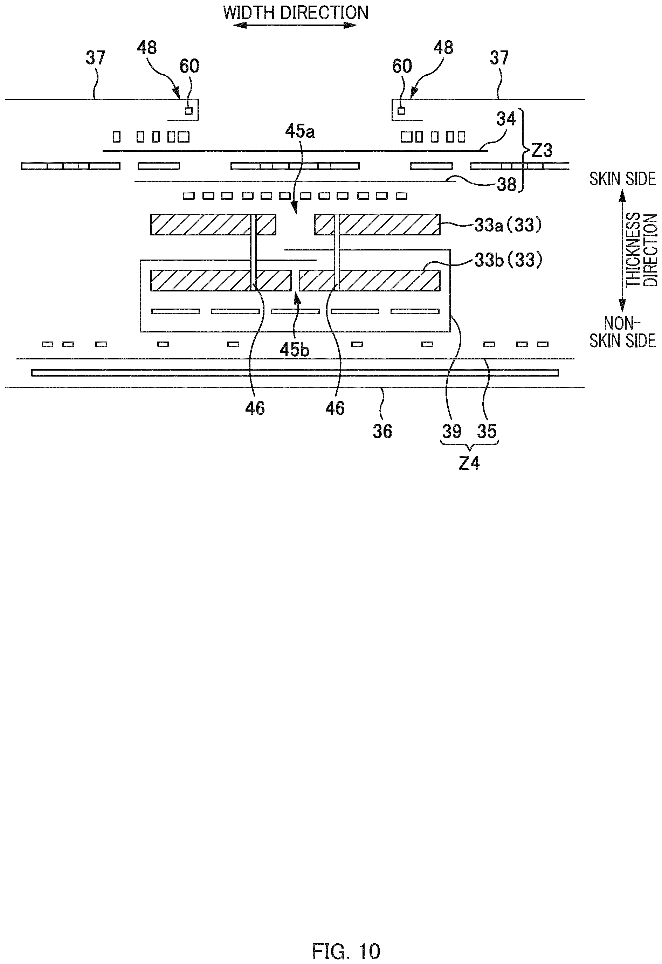

FIG. 8 is a schematic plan view of the absorbent pad 2 in the unfolded and stretched state, which is an absorbent article according to the second embodiment, as viewed from the skin surface side. FIG. 9 is an exploded perspective view of the main part of the absorbent pad 2 shown in FIG. 8. FIG. 10 is a cross-sectional view taken along line B-B shown in FIG. 8.

As shown in FIG. 8, similarly to the first embodiment, the absorbent pad 2 has the lengthwise direction, the width direction, and the thickness direction (not shown). Further, as shown in FIG. 10, the direction in which the constituent members of the absorbent pad 2 are overlaid on each other is referred to as the thickness direction. In the thickness direction, the side that is in contact with the wearer is called the skin side, and the opposite side is called the non-skin side. Note that the unfolded state and the stretched state of the absorbent pad 2 are the same as the definitions of the unfolded state and the stretched state in the first embodiment.

As shown in FIGS. 8 and 9, the absorbent pad 2 includes a top-surface sheet 34, a back-surface sheet 35, an absorbent body 33, an exterior sheet 36, and a pair of side sheets 37. The top-surface sheet 34 is a liquid-permeable sheet provided on the skin side in the thickness direction with respect to the absorbent body 33, and examples thereof include an air-through nonwoven fabric sheet, a spunbond nonwoven fabric sheet, and the like. The back-surface sheet 35 is a liquid-impermeable sheet provided on the non-skin side in the thickness direction with respect to the absorbent body 33, and examples thereof include a synthetic resin film and a hydrophobic SMS nonwoven fabric sheet and the like. Additionally, the top-surface sheet 34 and the back-surface sheet 35 contain thermoplastic resin (polyethylene (PE), polypropylene (PP), and the like).

The pair of side sheets 37 each are a sheet (e.g., nonwoven fabric sheet, and the like) located on the skin side of the top-surface sheet 34 and extending outward in the width direction from both side portions in the width direction. The exterior sheet 36 is a liquid-impermeable sheet located on the non-skin side in the thickness direction with respect to the back-surface sheet 5, and has a shape (that is, the external shape of the absorbent pad 2) in which the center portion in the lengthwise direction is narrowed inward in the width direction.

The absorbent body 33 is a member that is located between the top-surface sheet 34 and the back-surface sheet 35 in the thickness direction and that has liquid absorbency and liquid retaining properties. In the second embodiment, the absorbent body 33 includes the first absorbent core 33a that is located on the skin side, and the second absorbent core 33b that is located on the non-skin side of the first absorbent core 33a. For example, the first absorbent core 33a and the second absorbent core 33b can be obtained by molding liquid-absorbent fiber such as pulp containing superabsorbent polymer (SAP) into a predetermined shape. Specifically, in the present embodiment, the shape of the first absorbent core 33a is a substantially gourd shape or a substantially hourglass shape, which is elongated in the lengthwise direction and has a portion narrowed inward in the width direction, near the center in the lengthwise direction. On the other hand, the shape of the second absorbent core 33b has a substantially rectangular shape which is long in the lengthwise direction and short in the width direction in a plan view. In the second absorbent core 33b, the dimension in the lengthwise direction is shorter than the dimension of the first absorbent core 33a in the lengthwise direction, and the dimension in the width direction is shorter than the dimension of the first absorbent core 33a in the width direction. Therefore, both edges of the first absorbent core 33a in the lengthwise direction and the width direction are respectively located outside both edges of the second absorbent core 33b in the lengthwise direction and the width direction. Note that there is no particular limitation on the shape of the absorbent body 33.

Further, in the second embodiment, the first core-wrapping sheet 38 is provided on the skin side of the first absorbent core 33a, and a second core-wrapping sheet 39 is provided so as to warp the second absorbent core 33b from the non-skin side. Note that in the second embodiment, the first core-wrapping sheet 38 is arranged so as not to wrap the first absorbent core 33a. Further, the first and second core-wrapping sheets 38 and 39 contain thermoplastic resin in addition to pulp fiber as the liquid-absorbent fiber. Examples of the thermoplastic resin include the polyethylene (PE) and the polypropylene (PP). The details of the first and second core-wrapping sheets 38 and 39 will be described later.

Further, the absorbent pad 2 has leak-proof walls 48. The leak-proof walls 48 are configured to be able to rise up on the side of the wearer's skin surface, and are provided in pair on the left and right sides. As shown in FIG. 10, the leak-proof walls 48 are each formed as follow: an inner side portion of the side sheet 37 in the width direction is folded back to the non-skin side, and a leak-proof-wall elastic member 60 that stretches and contracts in the lengthwise direction is provided in the folded back portion. The leak-proof wall 48 can suppress the side leakage of excrement. In addition, in FIG. 8, one string-like leak-proof-wall elastic member 60 is arranged spacing on each side in the width direction, but the number of the leak-proof-wall elastic members 60 is not particularly limited.

In the present embodiment, in the absorbent body 33, the first absorbent core 33a has a slit 45a that is recessed, preferably penetrating, in the thickness direction from the skin side towards the non-skin side. The slit 45a is located on the front side with respect to the center of the absorbent pad 2 in the lengthwise direction, and extends in the lengthwise direction through the center of the absorbent body 33 in the width direction. The second absorbent core 33b has a slit 45b that is recessed, preferably penetrating, in the thickness direction from the skin side towards the non-skin side. The slit 45b is located on the front side with respect to the center of the absorbent pad 2 in the lengthwise direction, and extends in the lengthwise direction through the center of the absorbent body 33 in the width direction. The slit 45a and the slit 45b are formed so that at least portions thereof overlap in a plan view. Thereby, when excreted urine reaches the second absorbent core 33b via the slit 45a of the first absorbent core 33a, the urine can be drawn further into the second absorbent core 33b via the slit 45b. The dimensions (length) of the slits 45a and 45b in the lengthwise direction and the dimensions (width) of the slits 45a and 45b in the width direction in a plan view are adjusted as appropriate in consideration of the sizes of the first absorbent core 33a and the second absorbent core 33b. The width and length of the slits 45a and 45b are, for example, 5.0 to 50 mm and 50 to 300 mm.

In the present embodiment, the absorbent body 33 includes a pair of compressed portions 46. The pair of compressed portions 46 extend along the lengthwise direction on both outer sides of the slit 45a in the width direction. The pair of the compressed portions 46 are formed by compressing the first absorbent core 33a and the second absorbent core 33b in the thickness direction from the skin-side surface of the first absorbent core 33a. However, the pair of compressed portions 46 may be formed by compressing both the top-surface sheet 34 and the absorbent body 33. The pair of compressed portions 46 are each formed in a straight-line manner in a plan view, but the shape is not particularly limited. The pair of the compressed portions 46 can prevent urine from moving outward on the top-surface sheet 34 and leaking to the outside, and can make it likely to draw urine into the absorbent body 33.

### Recycled sheet, separate sheet, and composite sheet in second embodiment

Similarly to the first embodiment, the first core-wrapping sheet 38 and the second core-wrapping sheet 39 in the second embodiment are recycled sheets containing 15 wt% or more of the recycled pulp which is obtained by recycling used disposable absorbent articles (e.g., used disposable diapers and used absorbent pads) (hereinafter, the first core-wrapping sheet 38 is also referred to as a first recycled sheet, and the second core-wrapping sheet 39 is also referred to as a second recycled sheet) . The absorbent pad 2 includes a composite sheet in which the first recycled sheet and a separate sheet adjacent to the first recycled sheet in the thickness direction of the absorbent pad 2 are joined. Specifically, the separate sheet for the first core-wrapping sheet 38 (first recycled sheet) is the top-surface sheet 34, and the first core-wrapping sheet 38 and the top-surface sheet 34 are joined to form a first composite sheet Z3 (see FIG. 10). Further, the absorbent pad 2 includes a composite sheet in which the second recycled sheet and a separate sheet adjacent to the second recycled sheet in the thickness direction of the absorbent pad 2 are joined. Specifically, the separate sheet for the second core-wrapping sheet 39 (second recycled sheet) is the back-surface sheet 35, and the non-skin-side surface of the second core-wrapping sheet 39 and the back-surface sheet 35 are joined to form a second composite sheet Z4 (see FIG. 10). Therefore, the absorbent pad 2 has the first composite sheet Z3 that is a composite sheet placed on the skin side of the first absorbent core 33a in the thickness direction, and a second composite sheet Z4 that is a composite sheet placed on the non-skin side of the second absorbent core 33b in the thickness direction.

The effect exhibited by the first composite sheet Z3 is the same as that of the skin-side composite sheet Z1 of the first embodiment, and the effect exhibited by the second composite sheet Z4 is the same as that of the non-skin-side composite sheet Z2 of the first embodiment. Therefore, in the following description, different points will be explained in detail, and explanations of common functions and effects will be omitted.

In the present embodiment, the mass ratio of the superabsorbent polymer contained in the first absorbent core 33a is greater than the mass ratio of the superabsorbent polymer contained in the second absorbent core 33b. Here, the mass ratio of the superabsorbent polymer in each of the absorbent cores is the ratio of the mass of the superabsorbent polymer to the total mass of the absorbent core (that is, fiber aggregate mass + mass of superabsorbent polymer). Therefore, when absorbing excreted fluid, the first absorbent core 33a is more likely to swell than the second absorbent core 33b. The first core-wrapping sheet 38 of the first absorbent core 33a (first recycled sheet) is provided so as not to wrap around the first absorbent core 33a, and the second core-wrapping sheet 39 of the second absorbent core 33b (second recycled sheet) is provided so as to wrap around the second absorbent core 33b. Since the first core-wrapping sheet 38 of the first absorbent core 33a, which has a large amount of superabsorbent polymer (SAP), is made into a form that does not wrap around the first absorbent core 33a, this reduces the burden on the first core-wrapping sheet 38 of the first absorbent core 33a, which has a large amount of the superabsorbent polymer and thereby is more likely to swell, making it possible to reduce the risk that the first core-wrapping sheet 38 break.

In addition, in the present embodiment, the first absorbent core 33a is located on the skin side and the second absorbent core 33b is located on the non-skin side of the first absorbent core 33a. But the present invention is not limited to this, and the first absorbent core 33a may be located on the non-skin side and the second absorbent core 33b may be located on the skin side of the first absorbent core 33a. That is, the absorbent pad 2 may be configured to provide the absorbent core, which has a high mass ratio of the superabsorbent polymer, on the non-skin side.

In addition, in the case where the thermoplastic resin is contained in the top-surface sheet 34 (separate sheet) and the first core-wrapping sheet 38 as described above, for example, if compressed portions such as the above-described pair of compressed portions 46 are formed on the skin side of the top-surface sheet 34, the thermoplastic resin of the top-surface sheet 34 and the thermoplastic resin of the first core-wrapping sheet 38 are thermally-fused. This makes it possible to more firmly join the top-surface sheet 34 (separate sheet) and the first core-wrapping sheet 38 (first recycled sheet). Therefore, the strength of the recycled sheet in the first composite sheet Z3 can be increased.

In the case where the top-surface sheet 34 (separate sheet) and the first core-wrapping sheet 38 (first recycled sheet) are thermally-fused, it is desirable that the thermoplastic resin of the first recycled sheet and the thermoplastic resin of the separate sheet are the same type of the thermoplastic resin. Thermal-fusing of the same type of the thermoplastic resin makes it possible to increase the strength of the first core-wrapping sheet 38 (first recycled sheet) in the first composite sheet Z3.

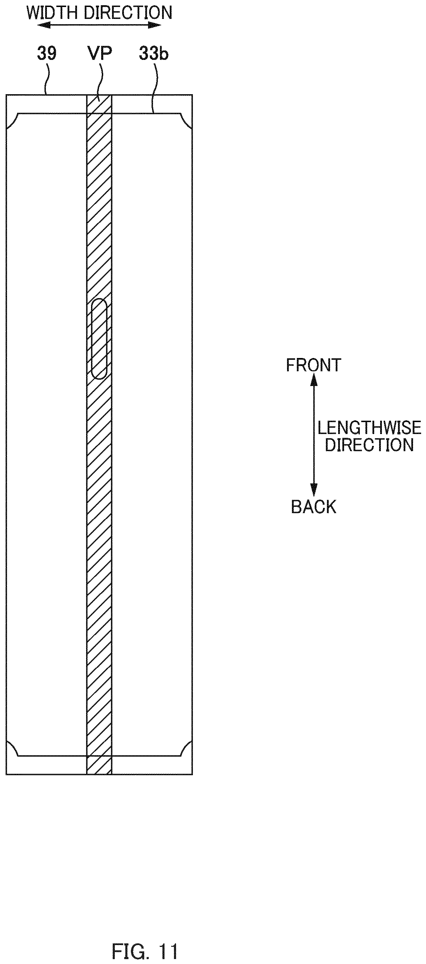

FIG. 11 is a plan view of the second absorbent core 33b and the second core-wrapping sheet 39 of the absorbent pad 2 in the unfolded state. In order to show the overlap of the second core-wrapping sheet 39, only the second absorbent core 33b and the second core-wrapping sheet 39 are shown for convenience. As shown in FIG. 11, at least a part of the second core-wrapping sheet 39 (second recycled sheet) has an overlapping region VP where portions of the second core-wrapping sheet 39 overlap in a plan view. In the overlapping region VP, the portions of the second core-wrapping sheet 39 are thermally-fused to each other. Since the second core-wrapping sheet 39 contains the thermoplastic resin, by thermally-fusing the portions of the second core-wrapping sheet 39, it is possible to increase the strength of the second core-wrapping sheet 39 (second recycled sheet) and to further reduce the risk of breakage.

### Others

The above embodiments are simply to facilitate understanding of the present disclosure and are not in any way to be construed as limiting the present disclosure. The present disclosure may variously be changed or altered without departing from its gist and encompass equivalents thereof.

In the first and second embodiments described above, the recycled sheet contains 15 wt% or more of the recycled pulp, but the present invention is not limited to this. The tensile test results shown in FIGS. 3 and 4 are the effects obtained when using a recycled sheet (skin-side core-wrapping sheet 8) in which the content of the recycled pulp is 15 wt%. Therefore, by making the content of the recycled pulp in the recycled sheet be less than 15 wt%, the recycled sheet can maintain the above-mentioned strength, and by using such a recycled sheet for the diaper 1, it is possible to reduce the risk of damage to the skin-side core-wrapping sheet 8, which is the recycled sheet, even when used for a long time.

In the first and second embodiments described above, the skin-side core-wrapping sheet 8, the non-skin-side core-wrapping sheet 9, the first core-wrapping sheet 38, and the second core-wrapping sheet 39 contain the recycled pulp that is obtained by recycling the disposable absorbent articles, but the present invention is not limited to this. For example, the absorbent core 3, the first absorbent core 33a, and the second absorbent core 33b may contain the recycled pulp, or another sheet material constituting the absorbent article may contain the recycled pulp.

### REFERENCE SIGNS LIST

1 diaper (absorbent article) (first embodiment)
1A front waist portion
1B crotch portion
1C back waist portion
2 absorbent pad (absorbent article) (second embodiment)
3 absorbent core
4 top-surface sheet (separate sheet)
5 back-surface sheet (separate sheet)
6 exterior sheet
7 side sheet
8 skin-side core-wrapping sheet (recycle sheet)
9 non-skin-side core-wrapping sheet (recycle sheet)
10 flap portion
11 base sheet
12 first fastening member
13 second fastening member
14 target portion
16 leg elastic member
17 inner leak-proof gather
18 outer leak-proof gather
20 application region
21 waist elastic member
22 waist gather
25 joining region
26 non-joining region
27 core joining region
33 absorbent body
33a first absorbent core
33b second absorbent core
34 top-surface sheet (separate sheet)
35 back-surface sheet (separate sheet)
36 exterior sheet
37 side sheet
38 first core-wrapping sheet (first recycled sheet)
39 second core-wrapping sheet (second recycled sheet)
45a slit
45b slit
46 a pair of compressed portions
48 leak-proof wall
60 leak-proof-wall elastic members
171 inner leak-proof elastic member
181 outer leak-proof elastic member
Z1 skin-side composite sheet (composite sheet)
Z2 non-skin-side composite sheet (composite sheet)
Z3 first composite sheet (composite sheet)
Z4 second composite sheet (composite sheet)

## Claims

1. An absorbent article comprising:
a recycled sheet containing 15 wt% or more of recycled pulp that is obtained by recycling a used absorbent article; and
a composite sheet in which the recycled sheet and a separate sheet that is adjacent to the recycled sheet in a thickness direction of the absorbent article are joined,
a tensile force per unit width at a timing when the recycled sheet starts to break in the composite sheet if the composite sheet is pulled in a fiber orientation direction of the recycled sheet
being higher than
a tensile force per unit width at a timing when the separate sheet starts to break if the separate sheet alone is pulled in the fiber orientation direction.

2. An absorbent article comprising:
a recycled sheet containing recycled pulp that is obtained by recycling a used absorbent article; and
a composite sheet in which the recycled sheet and a separate sheet that is adjacent to the recycled sheet in a thickness direction of the absorbent article are joined,
a content of the recycled pulp in the recycled sheet being less than 15 wt%,
a tensile force per unit width at a timing when the recycled sheet starts to break in the composite sheet if the composite sheet is pulled in a fiber orientation direction of the recycled sheet
is higher than
a tensile force per unit width at a timing when the separate sheet starts to break if the separate sheet alone is pulled in the fiber orientation direction.

3. The absorbent article according to claim 1 or 2, wherein
in the composite sheet,
the fiber orientation direction of the recycled sheet and the fiber orientation direction of the separate sheet are an identical direction, and
a maximum elongation of the separate sheet in the fiber orientation direction
is lower than
a maximum elongation in a direction perpendicular to the fiber orientation direction.

4. The absorbent article according to any one of claims 1 to 3, wherein
In the fiber orientation direction,
a tensile force per unit width when a length of the separate sheet after elongation is 105% of the length of the separate sheet before elongation
is higher than
a tensile force per unit width when the recycled sheet starts to break when pulled in the fiber orientation direction.

5. The absorbent article according to any one of claims 1 to 4, wherein
the recycled sheet contains a thermoplastic resin.

6. The absorbent article according to claim 5, wherein
the separate sheet contains a thermoplastic resin, and
the core-wrapping sheet and the separate sheet are thermally-fused.

7. The absorbent article according to claim 6, wherein
the thermoplastic resin of the recycled sheet and the thermoplastic resin of the separate sheet are an identical type of thermoplastic resin.

8. The absorbent article according to any one of claims 1 to 7, wherein
in the composite sheet, the recycled sheet and the separate sheet are joined with a hot-melt adhesive.

9. The absorbent article according to claim 8, wherein
the hot-melt adhesive is applied in a planar manner to at least a part.

10. The absorbent article according to claim 8, wherein
the composite sheet has a non-joined region where the recycled sheet and the separate sheet are not joined with the hot-melt adhesive.

11. The absorbent article according to claim 10, wherein
in an unfolded state,
the fiber orientation direction is a lengthwise direction of the absorbent article, and
a direction perpendicular to the lengthwise direction is a width direction, and
letting a region in the composite sheet where the recycled sheet and the separate sheet are joined with the hot-melt adhesive be a joining region,
a total length of the joining region in the width direction is longer than a total length of the non-joining regions in the width direction.

12. The absorbent article according to any one of claims 8 to 11, wherein
in an unfolded state,
the fiber orientation direction is a lengthwise direction of the absorbent article, and
a direction perpendicular to the lengthwise direction is a width direction, and
a length where the hot-melt adhesive is provided continuously in the composite sheet is longer in the lengthwise direction than in the width direction.

13. The absorbent article according to claim 8 or 9, wherein
at least a part of a joining region where the recycled sheet and the separate sheet are joined with the hot-melt adhesive is provided over an entire range of the recycled sheet in the lengthwise direction.

14. The absorbent article according to any one of claims 1 to 13, wherein
the absorbent article has an absorbent core,
the absorbent article has
a skin-side composite sheet that is the composite sheet placed on the skin side in the thickness direction of the absorbent core, and
a non-skin-side composite sheet that is the composite sheet placed on the non-skin side in the thickness direction of the absorbent core,
the recycled sheet of the skin-side composite sheet is a skin-side core-wrapping sheet that is located adjacent to the absorbent core on a skin side, and
the recycled sheet of the non-skin-side composite sheet is a non-skin-side core-wrapping sheet that is located adjacent to the absorbent core on a non-skin side.

15. The absorbent article according to claim 14, wherein
the separate sheet of the skin-side composite sheet is a liquid-permeable sheet member,
the separate sheet of the non-skin-side composite sheet is a liquid-impermeable sheet member, and
a tensile force per unit width at a timing when the recycled sheet starts to break in the non-skin-side composite sheet if the non-skin-side composite sheet is pulled in a fiber orientation direction of the recycled sheet
is higher than
a tensile force per unit width at a timing when the recycled sheet starts to break in the skin-side composite sheet if the skin-side composite sheet is pulled in the fiber orientation direction of the recycled sheet.

16. The absorbent article according to claim 14 or 15, wherein
the separate sheet of the non-skin-side composite sheet is located adjacent to the non-skin-side core-wrapping sheet on the non-skin side,
the non-skin-side composite sheet has a non-joined region where the non-skin-side core-wrapping sheet and the separate sheet are not joined with a hot-melt adhesive,
the absorbent core and the non-skin-side core-wrapping sheet have a core joining region where the absorbent core and the non-skin-side core-wrapping sheet are joined to each other with the hot-melt adhesive, and
the non-joining region has a portion that overlaps the core joining region in a plan view of the absorbent article that is in an unfolded state.

17. The absorbent article according to any one of claims 1 to 13, wherein
the absorbent article includes
a first absorbent core that contains at least a superabsorbent polymer, and
a second absorbent core that contains at least a superabsorbent polymer,
a mass ratio of the superabsorbent polymer contained in the first absorbent core is greater than a mass ratio of the superabsorbent polymer contained in the second absorbent core,
a first recycled sheet that is the recycled sheet is provided as a core-wrapping sheet that is arranged so as not to wrap the first absorbent core, and
a second recycled sheet that is the recycled sheet is provided as a core-wrapping sheet that is arranged so as to wrap the second absorbent core.

18. The absorbent article according to claim 17, wherein
the recycled sheet contains a thermoplastic resin,
at least a part of the second recycled sheet has an overlapping region where portions of the recycled sheet overlap each other, in a plan view of the absorbent article that is in the unfolded state, and
in the overlapping region, the portions of the recycled sheet are thermally-fused to each other.
